(19) Europäisches Patentamt
European Patent Office
Office européen des brevets

(11) Veröffentlichungsnummer : **0 027 928**
**B1**

(12) # EUROPÄISCHE PATENTSCHRIFT

(45) Veröffentlichungstag der Patentschrift : **09.03.83**

(51) Int. Cl.³ : **C 07 D401/12**, C 07 D401/14, A 61 K 31/445

(21) Anmeldenummer : **80106069.0**

(22) Anmeldetag : **07.10.80**

(54) **Piperidinderivate von 4,5-Dialkyl-3-hydroxy-pyrrol-2-carbonsäureestern, ihre Herstellung und diese enthaltende pharmazeutische Zubereitungen.**

(30) Priorität : **13.10.79 DE 2941597**

(43) Veröffentlichungstag der Anmeldung : **06.05.81 Patentblatt 81/18**

(45) Bekanntmachung des Hinweises auf die Patenterteilung : **09.03.83 Patentblatt 83/10**

(84) Benannte Vertragsstaaten :
**AT BE CH DE FR GB IT LI LU NL SE**

(56) Entgegenhaltungen :
**DE A 1 695 111**
**DE A 1 720 033**
**FR M 1 527**
**US A 3 860 609**

(73) Patentinhaber : **BASF Aktiengesellschaft**
**Carl-Bosch-Strasse 38**
**D-6700 Ludwigshafen (DE)**

(72) Erfinder : **Frickel, Fritz-Frieder, Dr.**
**Silvanerweg 7**
**D-6705 Deidesheim (DE)**
Erfinder : **Franke, Albrecht, Dr.**
**Mandelring 11**
**D-6706 Wachenheim (DE)**
Erfinder : **Von Philipsborn, Gerda, Dr.**
**Naechstenbacher Weg 35**
**D-6940 Weinheim (DE)**
Erfinder : **Mueller, Claus D., Dr.**
**Odenwaldring 84**
**D-6806 Viernheim (DE)**
Erfinder : **Lenke, Dieter, Dr.**
**Kekuleplatz 1**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 027 928 B1

Piperidinderivate von 4,5-Dialkyl-3-hydroxy-pyrrol-2-carbonsäureestern, ihre Herstellung und diese enthaltende pharmazeutische Zubereitungen

Die Erfindung betrifft substituierte Piperidinalkyläther von 4,5-Dialkyl-3-hydroxy-2-pyrrol-2-carbonsäureestern und ihre physiologisch verträglichen Säureadditionssalze, Verfahren zu ihrer Herstellung und diese enthaltende pharmazeutische Zubereitungen, die bei der Behandlung von Herzrhythmusstörungen verwendet werden können.

In der DE-A- 17 20 033 werden Indolverbindungen, die in 3-Stellung einen basischen Ätherrest, wobei die basische Gruppe ein Piperidylrest sein kann, beschrieben. Für diese Verbindungen wird eine spasmolytische Wirksamkeit und eine Wirkung gegen Asthma angegeben.

In der DE-A- 26 30 152 werden beispielsweise Derivate des 1-Phenoxy-propanol-2 mit einem 4-(2-Pyridyl)-piperidin-4-ol-Rest mit antiarrhythmische Eigenschaften beschrieben. Dem Fachmann ist bekannt, daß die bisher eingesetzten Mittel zur Bekämpfung von Herzrhythmusstörungen nicht immer befriedigen und diese häufig eine zu geringe therapeutische Breite aufweisen.

Es wurde gefunden, daß Verbindungen der allgemeinen Formel I

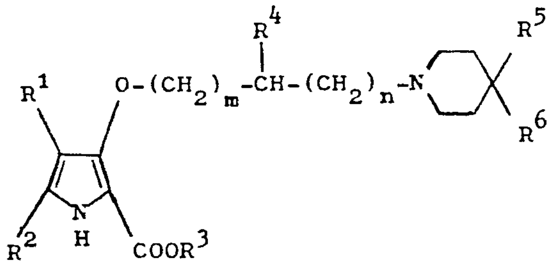

(I)

in der $R^1$ einen Alkylrest mit 1 bis 4 C-Atomen, insbesondere Methyl, $R^2$ Methyl, $R^3$ Methyl oder Äthyl, $R^4$ ein Wasserstoffatom oder eine Hydroxylgruppe, $R^5$ eine Hydroxylgruppe und $R^6$ α-Pyridyloder ein Phenylring, der ggf. durch ein Fluor-, Chlor oder Bromatom substituiert ist, bedeuten, wobei m und n 1 oder 2 wenn $R^4$ ein H-Atom darstellt, zusätzlich m 1 und n 0 sind, und ihre physiologisch verträglichen Säureadditionssalze wertvolle pharmakologische Eigenschaften aufweisen.

Die erfindungsgemäßen Verbindungen werden hergestellt, indem man ein Carboalkoxypyrrol der Formel II

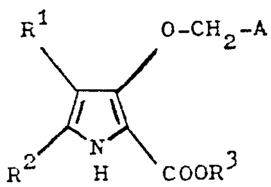

(II)

in der $R^1$, $R^2$ und $R^3$ die für Formel I angegebenen Bedeutungen haben und A den Rest

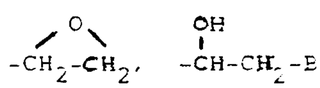 oder $-(CH_2)_l-B$,

wobei B für eine nukleofuge Abgangsgruppe und l für eine ganze Zahl von 1 bis 4 stehen, bedeutet, mit einem Piperidinderivat der allgemeinen Formel III

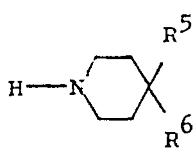

(III)

in der $R^5$ und $R^6$ die für die Formel I angegebenen Bedeutungen haben, zweckmäßigerweise in einem Lösungsmittel und ggf. in Gegenwart eines säurebindenden Mittels in an sich bekannter Weise umsetzt und die erhaltene Verbindung ggf. in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120 °C, d. h. bei Raumtemperaturen oder bei höheren Temperaturen, zweckmäßig bei Temperaturen von 50 bis 120 °C, durchgeführt. Die Umsetzungen können unter Atmosphärendruck oder in einem geschlossenen Gefäß unter erhöhtem Druck ggf. unter Erwärmung auf den angegebenen Temperaturbereich durchgeführt werden.

Die Ausgangsverbindungen können direkt, d. h. ohne Zugabe eines Verdünnungs- oder Lösungsmittels umgesetzt werden. Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines aromatischen Kohlenwasserstoffes, wie Benzol oder eines Alkylbenzols, wie Toluol oder Xylol, oder eines gesättigten aliphatischen Kohlenwasserstoffes, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, oder von Dimethylsulfoxid oder in Gegenwart von Wasser oder in Mischungen der genannten Lösungsmittel, durchgeführt.

Bevorzugte Lösungsmittel bei der Umsetzung eines Epoxids der Formel (II), beispielsweise von 1-(2-Carbomethoxy-4,5-dimethyl-pyrrol-3-oxy)-2,3-epoxypropan ; 1-(2-Carboäthoxy-4,5-dimethyl-pyrrol-3-oxy)-2,3-epoxypropan oder 1-(2-Carboäthoxy-5-methyl-4-n-butyl-pyrrol-3-oxy)-2,3-epoxypropan, mit einem Piperidinderivat der allgemeinen Formel (III) sind niedere Alkohole, insbesondere Isopropanol, wobei die Umsetzung bevorzugt bei Temperaturen von 50 bis 120 °C und bei Normaldruck durchgeführt wird.

Bei nukleophilen Substitutionen eines Restes B in einer Verbindung der Formel (II) mit einem Rest $-(CH_2)_l-B$, beispielsweise von 1-(2-Carboäthoxy-4,5-dimethyl-pyrrol-3-oxy)-3-chlor-propan oder 1-(2-Carbomethoxy-4,5-dimethyl-pyrrol-3-oxy)-4-chlor-butan, sind als Lösungsmittel ein niederes aliphatisches Keton, wie Aceton, Diäthylketon, Methylisopropylketon oder Methylisobutylketon, ein cyclischer gesättigter Äther, insbesondere Tetrahydrofuran oder Dioxan, oder ein Dialkylformamid, wie Dimethylformamid, und Temperaturen von 90 bis 180 °C, bevorzugt. Gegebenenfalls wird die Reaktion in Gegenwart einer katalytischen Menge Natrium- oder Kaliumjodid durchgeführt.

Es sei erwähnt, daß als Ausgangsverbindung der Formel II gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmäßigen Ausführungsform zur nukleophilen Substitution des Restes B durch das verwendete Piperidinderivat wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin oder ein Trialkylamin, wie Trimethylamin oder Triäthylamin. Von den Alkaliverbindungen kommen insbesondere die des Natriums oder Kaliums in Betracht. Dabei wird die Base in stöchiometrischer Menge oder in geringem Überschuß verwendet. Gegebenenfalls ist es zweckmäßig, das zur Umsetzung verwendete Piperidinderivat (III) in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel (II) können durch Alkylierung des der Verbindung zugrundeliegenden 3-Hydroxy-pyrrolverbindung, beispielsweise von 3-Hydroxy-4,5-dimethyl-2-pyrrolcarbonsäureäthylester oder 4-Butyl-3-hydroxy-5-methyl-2-pyrrolcarbonsäureäthylester mit einem Epihalogenhydrin, einem α,ω-Dihalogen-2-propanol oder α,ω-Dihalogenpropan erhalten werden. Als Epihalogenhydrine kommen Epichlorhydrin, Epibromhydrin und Epijodhydrin, als α,ω-Dihalogen-2-propanole kommen insbesondere 1,3-Dichlor-2-propanol und 1,3-Dibrom-2-propanol und als α,ω-Dihalogenpropane insbesondere 1,3-Chlorbrompropan ; 1,3-Dichlorpropan, 1,3-Dibrompropan, 1,2-Chlorbromäthan oder 1,4-Chlorbrombutan in Betracht.

Die Herstellung der benötigten Hydroxy-pyrrol-carbonsäureester kann Liebigs Annalen der Chemie 1976, Seiten 384 bis 386, entnommen werden oder kann, so beispielsweise 3-Hydroxy-4,5-dimethyl-2-pyrrolcarbonsäuremethylester analog zu dem in Liebigs Annalen der Chemie, Band 736, Seiten 1 bis 15 (1970) beschriebenen Verfahren erfolgen.

Die Alkylierung der 2-Carbolkoxy-3-hydroxy-pyrrole zur Herstellung der Ausgangsverbindungen der Formel (II) werden zweckmäßigerweise bei Temperaturen von 50 bis 120 °C und unter Normaldruck oder in einem geschlossenen Gefäß unter erhöhtem Druck durchgeführt. Die Umsetungen werden zweckmäßigerweise in einem inerten Verdünnungs- oder Lösungsmittel, z. B. einen niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem niederen Alkylacetat, wie Methyl-, Äthyl- oder

Propylacetat, einem Dialkylformamid, wie Dimethylformamid oder Diäthylformamid, oder Dimethylsulfoxid oder mit überschüssigem Alkylierungsmittel als Verdünnungs- oder Lösungsmittel durchgeführt.

Bevorzugt werden die Umsetzungen in Gegenwart einer Base als säurebindendem Mittel vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem Überschuß verwendet werden.

Bevorzugt werden die 2-Carboalkoxy-3-hydroxy-pyrrole mit Epibromhydrin ; 1,3-Dibrompropanol-2 ; 1,3-Dibrompropan ; 1,4-Bromchlorbutan oder 1,2-Bromchloräthan in einem niederen aliphatischen Keton, insbesondere Aceton oder Methyl-isobutylketon, in Gegenwart von mindestens einem Moläquivalent Base, insbesondere Kaliumcarbonat, bezogen auf das Alkylierungsmittel, bei Temperaturen von 50 bis 80 °C umgesetzt.

Weiterhin sei erwähnt, daß die Ausgangsverbindungen der Formel (II) mit einer Epoxygruppe oder mit Halogenhydrinstruktur durch einfache Säure-Base-Reaktion ineinander umgewandelt werden können. So läßt sich ein 1-(2-Carboäthoxy-4,5-dimethyl-pyrrol-3-oxy)-2,3-epoxypropan mit der entsprechenden Halogenwasserstoffsäure in 1-(2-Carboäthoxy-4,5-dimethylpyrrol-3-oxy)-3-halogen-propanol-2 überführen, wobei als Verdünnungs- oder Lösungsmittel neben an sich üblichen Solventien vorzugsweise ein aliphatischer oder cyclischer Äther, wie Diäthyläther, Tetrahydrofuran oder Dioxan, oder ein niederer Alkohol, wie Methanol, Äthanol oder Propanol verwendet werden. Andererseits können die 1-(2-Carboäthoxy-4,5-dimethyl-pyrrol-3-oxy)-3-halogen-propanol-2-Verbindungen, insbesondere das 1-(2-Carboäthoxy-4,5-dimethyl-pyrrol-3-oxy)-3-chlor-propanol-2 und das 1-(2-Carboäthoxy-4,5-dimethylpyrrol-3-oxy)-3-brom-propanol-2 mit einer Base, wie einem Alkalimetall-hydroxid, -carbonat, -hydrogencarbonat, -alkoholat oder -hydrid, einem tertiär-organischen Amin, wie Pyridin, Piperidin oder einem tertiären aliphatischen Amin, wie Trimethylamin oder Triäthylamin, in 1-(2-Carboäthoxy-4,5-dimethylpyrrol-3-oxy)-2,3-epoxypropan umgewandelt werden.

Diese Reaktionen können bei Raumtemperaturen durchgeführt werden oder durch Wärmezufuhr, z. B. durch Erwärmen auf 60 bis 120 °C, beschleunigt oder abgeschlossen werden.

Die Reaktion kann unter Atmosphärendruck oder in einem geschlossenen Gefäß unter erhöhtem Druck, gegebenenfalls bei gleichzeitigem Erwärmen, durchgeführt werden. Die Ausgangsstoffe für diese Umwandlung können zuvor isoliert oder im Reaktionsgemisch erzeugt und ohne weitere Isolierung und Reinigung unmittelbar weiterverarbeitet werden.

Die erfindungsgemäßen Verbindungen der Formel I, die eine Hydroxygruppe in der aliphatischen Seitenkette tragen, weisen ein Chiralitätszentrum auf und werden als Racemate erhalten, die durch bekannte Methoden, beispielsweise durch Bildung diastereomerer Salze mit optisch aktiven Hilfssäuren, wie Dibenzoylweinsäure, Campher-10-Sulfonsäure, Ditholuylweinsäure oder 3-Brom-campher-8-sulfonsäure, in die optisch aktiven Antipoden getrennt werden können.

Die erhaltenen erfindungsgemäßen Verbindungen werden gegebenenfalls nach bekannter Verfahrensweise in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure und als organische Säure beispielsweise Oxalsäure, Maleinsäure, Fumarsäure, Milchsäure, Weinsäure, Apfelsäure, Zitronensäure, Salicylsäure, Adipinsäure oder Benzoesäure oder können aus Fortschritte der Arzneimittelforschung Band *10*, Seite 224 bis 225, Birkhäuser-Verlag, Basel und Stuttgart, 1966 oder dem Journal of Pharmaceutical Science, Volume 66, Seiten 1 bis 5 (1977) entnommen werden.

Die erfindungsgemäßen Verbindungen und ihre physiologisch verträglichen Säureadditionssalze weisen wertvolle pharmakologische Eigenschaften auf. Sie sind stark antiarrhythmisch wirksam und daher insbesondere zur Pharmakotherapie von Herzrhythmusstörungen geeignet.

Zur Bestimmung der antiarrhythmischen Wirksamkeit werden die Substanzen Ratten (Stamm : Sprague Dawley, Gewicht 200 bis 250 g) 45 min vor Beginn der Narkose oral appliziert.

Die Tiere werden mit Thiobutabarbital-Natrium (100 mg/kg intraperitoneal, i. p.) narkotisiert. Als arrhythmogene Substanz dient Aconitin, das 60 min nach Substanzapplikation intravenös (i. v.) infundiert wird (Dosierungsgeschwindigkeit : 0,005 mg/kg × min). Bei nicht behandelten Tieren (N = 52) treten nach 2,74 = 0,07 min im Elektrokardiogramm (EKG) Arrhythmien auf, deren Eintritt durch Antiarrhythmica dosisabhängig verzögert werden kann.

Aus der linearen Beziehung zwischen log Dosis (mg/kg) der Prüfsubstanz und der relativen Verlängerung der Aconitininfusionsdauer (Δ %) wird die Dosis bestimmt, welche die Infusionsdauer um 50 % verlängert (ED 50 %).

Zur weiteren Charakterisierung der Substanzen wird aus der in den Experimenten verwendeten dezimalgeometrischen Dosenfolge (Faktor $\sqrt[3]{10}$) die antiarrhythmische Wirkung der maximal tolerierten Dosis bestimmt. Ferner wird die Dosis ermittelt, bei welcher toxische Symptome (Veränderungen des Ausgangs-EKG, Cyanose, Krämpfe) auftreten. Als Vergleichssubstanz dient das bekannte hochwirksame Antiarrhythmicum Prajmalium (N-Propylajmalin).

Die Tabelle 1 zeigt, daß die untersuchten Verbindungen an der Aconitinarrhythmie der Ratte entweder ebenso antiarrhythmisch wirksam sind wie Prajmalium (Beispiele 2, 10, 12) oder dieses

übertreffen (Beispiele 5, 9 und 14). Ein weiterer Vorteil ist die im Vergleich mit Prajmalium höhere Wirkung bei der Applikation der höchsten tolerierten Dosis. Prajmalium verlängert die Aconitininfusionsdauer um maximal 174 %. Die Beispiele 1, 9, 10 und 14 bewirken eine maximale Zunahme um 324, 347, 308 und 302 %.

Die toxischen Dosen der erfindungsgemäßen Substanzen liegen mit Ausnahme des gleich toxischen Beispiels 5 höher als die des Prajmalium. Die Zunahme der Verträglichkeit bei vergleichsweise entsprechender oder höherer antiarrhythmischer Aktivität hat bei den erfindungsgemäßen Verbindungen eine Vergrößerung der therapeutischen Breite — als Quotient aus der toxischen und der antiarrhythmisch wirksamen Dosis (ED 50 %) — zur Folge.

Bei Prajmalium traten bereits toxische Symptome auf, wenn das 4,3 fache der wirksamen Dosis verabfolgt wird. Dagegen sind die toxischen Dosen bei den erfindungsgemäßen Substanzen 5,8 (Beispiel 5) bis 16 mal (Beispiel 9) größer als die wirksamen.

Tabelle 1

Antiarrhythmische Wirkung und Toxizität an der Ratte. Appl. : per os

| Substanz | Antiarrhythmische Wirkung an der Aconitinarrhythmie | | | | | | |
|---|---|---|---|---|---|---|---|
| | Wirksame Dosis mg/kg | | Maximale Wirkung[3] | | | Toxizität | |
| Beispiel | ED 50 %[1] | R. W.[2] | Dosis | Δ %[4] | R. M. W.[5] | Dosis[6] | Q[7] |
| 1 | 7,80 | 0,64 | 46,4 | 324 | 1,86 | 100 | 18 |
| 2 | 6,23 | 0,80 | 21,5 | 170 | 0,98 | 46,4 | 7,5 |
| 5 | 3,68 | 1,36 | 10 | 152 | 0,87 | 21,5 | 5,8 |
| 6 | 13,5 | 0,37 | 46,4 | 307 | 1,76 | 100 | 7,4 |
| 9 | 2,99 | 1,67 | 21,5 | 347 | 1,99 | 46,4 | 16 |
| 10 | 5,54 | 0,90 | 21,5 | 308 | 1,77 | 46,4 | 8,4 |
| 12 | 5,93 | 0,84 | 21,5 | 149 | 0,86 | 46,4 | 7,8 |
| 14 | 3,38 | 1,48 | 21,5 | 302 | 1,74 | 46,4 | 14 |
| 15 | 9,85 | 0,51 | 46,4 | 173 | 0,99 | 100 | 10 |
| Prajmalium | 4,99 | 1,00 | 10 | 174 | 1,00 | 21,5 | 4,1 |

1) Dosis, welche die Aconitininfusionsdauer um 50 % verlängert.
2) R. W. = Relative Wirksamkeit. Prajmalium = 1,00.
3) Wirkung der maximal tolerierten Dosis.
4) Verlängerung der Aconitininfusionsdauer Δ %.
5) R. M. W. = Relative maximale Wirkung. Prajmalium = 1,00.
6) Dosis, nach deren Appl. die ersten toxischen Symptome beobachtet werden.
7) $Q = \frac{\text{Toxische Dosis}}{\text{ED 50 \%}}$

Gegenstand der vorliegenden Erfindung sind demnach auch therapeutische Mittel oder Zubereitungen, die neben üblichen Träger- und Verdünnungsmitteln eine Verbindung der Formel I oder deren physiologisch verträgliches Säureadditionssalz als Wirkstoff enthalten.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Inkektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel, wie Maisstärke oder Alginsäure, Bindemittel, wie Stärke oder Gelatine, Gleitmittel, wie Magnesiumstearat oder Talk, und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi

arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können außerdem Suspendierstoffe, die Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfeten oder Polyäthylenglykol bzw. deren Derivaten herstellen.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 100,-vorzugsweise 10 bis 80 mg.

Die vorliegende Erfindung wird durch die nachfolgenden Ausführungsbeispiele näher erläutert.

## I. Herstellung von Ausgangsverbindungen

### Beispiel I

3-Hydroxy-4,5-dimethyl-2-pyrrolcarbonsäureäthylester

Zu einer gut gerührten Lösung aus 35 g Natrium in 1,5 Liter Äthanol tropft man unter Stickstoff 264 g 3-(Äthoxycarbonylmethylamino)-2-methylcrotonsäureäthylester in 400 ml Äthanol. Nach dreistündigem Sieden gibt man zur abgekühlten Lösung erst 600 ml Wasser, dann 150 ml konzentrierte Salzsäure. Nach sechsstündigem Stehen bei −10 °C werden 165 g 3-Hydroxy-4,5-dimethyl-2-pyrrolcarbonsäureäthylester vom Schmp. 111 bis 113 °C erhalten.

$C_9H_{13}NO_3$ (183,1)
Ber.: 59,0 C 7,1 H 7,7 N
Gef.: 59,2 C 7,2 H 7,5 N

### Beispiel II

3-Hydroxy-4,5-dimethyl-2-pyrrolcarbonsäuremethylester

Gemäß Beispiel 1 werden aus 5,7 g Natrium in 250 ml Methanol und 40 g 3-(Methoxycarbonylmethylamino)-2-methylcrotonsäure-methylester in 90 ml Methanol 28,1 g 3-Hydroxy-4,5-dimethyl-2-pyrrolcarbonsäuremethylester vom Schmp. 170 bis 172 °C erhalten.

$C_8H_{11}NO_3$ (169,1)
Ber.: 56,8 C 6,6 H 8,3 N
Gef.: 57,3 C 6,6 H 8,4 N

### Beispiel III

3-(Methoxycarbonylmethylamino)-2-methylcrotonsäuremethylester

Zu einer gut gerührten Suspension von 50 g Glycinmethylesterhydrochlorid in 20 ml Methanol werden 72 g einer 30 %igen Natriummethylat-Lösung in Methanol zugegeben und nach 15 Minuten 58 g 2-Methylacetessigsäureäthylester. Anschließend wird 8 Stunden auf Rückflußtemperatur erhitzt. Das auf Raumtemperatur abgekühlte Reaktionsgemisch wird mit 400 ml Äther verdünnt und 2 mal mit je 300 ml Wasser ausgeschüttelt. Die organische Phase wird über Magnesiumsulfat getrocknet, im Vakuum eingeengt und der Rohaustrag fraktioniert destilliert: 47,6 g 3-(Methoxycarbonylamino)-2-methylcrotonsäuremethylester von Sdp. 108 bis 110 °C bei 0,2 Torr.

$C_9H_{15}NO_4$ (201,1)
Ber.: 53,7 C 7,5 H 7,0 N
Gef.: 53,9 C 7,5 H 6,9 N

### Beispiel IV

1-(2-Carboäthoxy-4,5-dimethyl-pyrrol-3-oxy)-2,3-epoxypropan

100 g 2-Carboäthoxy-3-hydroxy-4,5-dimethylpyrrol, 150 g Epibromhydrin und 152 g trockenes

Kaliumcarbonat werden in 500 ml Aceton 16 Stunden auf Rückflußtemperatur erhitzt. Nach dem Erkalten gießt man die gesamte Reaktionsmasse auf 3 Liter Eiswasser, extrahiert mit Äther, wäscht die gesammelten Extrakte mit 2 N Natronlauge und Wasser und trocknet über Natriumsulfat. Der nach dem Abdestillieren von Äther und überschüssigem Epibromhydrin verbleibende Eindampfrückstand wird mit Heptan extrahiert. Man erhält 122 g 1-(2-Carboäthoxy-4,5-dimethyl-pyrrol-3-oxy)-2,3-epoxypropan vom Schmp. 70 bis 71 °C.

$C_{12}H_{17}NO_4$ (239,1)
Ber.: 60,3 C 7,1 H 5,9 N
Gef.: 60,1 C 7,0 H 6,0 N

Beispiel V

1-(2-Carbomethoxy-4,5-dimethyl-pyrrol-3-oxy)-2,3-epoxypropan

Gemäß Beispiel IV werden aus 17 g 2-Carbomethoxy-3-hydro-4,5-dimethylpyrrol, 20 g Epibromhydrin und 28 g trockenem Kaliumcarbonat in 100 ml Aceton 18,5 g 1-(2-Carbomethoxy-4,5-dimethyl-pyrrol-3-oxy)-2,3-epoxypropan vom Schmp. 85 bis 87 °C erhalten.

$C_{11}H_{15}NO_4$ (225,1)
Ber.: 58,7 C 6,7 H 6,2 N
Gef.: 58,5 C 6,7 H 6,5 N

Beispiel VI

1-(2-Carboäthoxy-4-butyl-5-methyl-pyrrol-3-oxy)-2,3-epoxypropan

Gemäß Beispiel IV werden aus 35 g 2-Carboäthoxy-3-hydroxy-4-butyl-5-methyl-pyrrol, 32 g Epibromhydrin und 41 g trockenem Kaliumcarbonat in 100 ml N,N-Dimethylformamid durch 8 stündiges Erwärmen auf 50 °C 41 g 1-(2-Carboäthoxy-4-butyl-5-methyl-pyrrol-3-oxy)-2,3-epoxypropan vom Schmp. 125 bis 127 °C erhalten.

$C_{15}H_{23}NO_4$ (281,2)
Ber.: 64,1 C 8,2 H 5,0 N
Gef.: 64,4 C 8,1 H 5,3 N

Beispiel VII

1-(2-Carbomethoxy-4,5-dimethyl-pyrrol-3-oxy)-3-chlorpropan

12 g 3-Hydroxy-4,5-dimethyl-2-pyrrolcarbonsäuremethylester, 13,8 1,3-Bromchlorpropan und 13 g wasserfreies Kaliumcarbonat werden in 50 ml N,N-Dimethylformamid 18 Stunden auf 50 °C erhitzt. Nach dem Erkalten wird abfiltriert und der Filterrückstand mit Aceton nachgewaschen. Das Filtrat wird zwischen Wasser/Methylenchlorid verteilt, die organische Phase mehrmals mit Wasser gewaschen, über Natriumsulfat getrocknet und im Vakuum eingedampft. Der verbleibende Rückstand wird aus Methanol unter Verwendung von Aktivkohle umkristallisiert. Es verbleiben 9 g 1-(2-Carbomethoxy-4,5-dimethyl-pyrrol-3-oxy)-3-chlorpropan vom Schmp. 117 bis 120 °C.

$C_{11}H_{16}NO_3Cl$ (245,5)
Ber.: 53,8 C 6,6 H 5,7 N 14,4 Cl
Gef.: 53,9 C 6,7 H 5,9 N 14,7 Cl

Beispiel VIII

1-(2-Carbomethoxy-4,5-dimethylpyrrol-3-oxy)-2-chloräthan

Gemäß Beispiel VII erhält man aus 12 g 3-Hydroxy-4,5-dimethyl-3-pyrrol-carbonsäuremethylester, 14, 2 g 1,2-Bromchloräthan und 13 g wasserfreiem Kaliumcarbonat 9,5 g 1-(2-Carbomethoxy-4,5-dimethylpyrrol-3-oxy)-2-chloräthan vom Schmp. 122 bis 123 °C.

$C_{10}H_{14}NO_3Cl$ (231,5)
Ber.: 51,8 C 61, H 6,1 N 15,3 Cl
Gef.: 51,3 C 6,0 H 6,0 N 15,6 Cl

Beispiel IX

1-(2-Carbomethoxy-4,5-dimethyl-pyrrol-3-oxy)-4-chlorbutan

Gemäß Beispiel VII erhält man aus 12 g 3-Hydroxy-4,5-dimethyl-2-pyrrol-carbonsäuremethylester, 15, 5 g 1,4-Bromchlorbutan und 12,3 g wasserfreiem Kaliumcarbonat 5,7 g 1-(2-Carbomethoxy-4,5-dimethyl-pyrrol-3-oxy)-4-chlorbutan vom Schmp. 74 bis 76 °C.

$C_{12}H_{18}NO_3Cl$ (259,5)
Ber. : 55,5 C 7,0 H 5,4 N 13,7 Cl
Gef. : 55,9 C 6,8 H 5,2 N 13,2 Cl

Beispiel X

1-(2-Carboäthoxy-4,5-dimethyl-pyrrol-3-oxy)-4-chlorbutan

Gemäß Beispiel VII erhält man aus 18,3 g 3-Hydroxy-4,5-dimethyl-2-pyrrolcarbonsäureäthylester, 22, 3 g 1,4-Bromchlorbutan und 18 g wasserfreiem Kaliumcarbonat 12,5 g 1-(2-Carboäthoxy-4,5-dimethyl-pyrrol-3-oxy)-4-chlorbutan vom Schmp. 58 bis 60 °C.

$C_{13}H_{20}NO_3Cl$ (273,5)
Ber. : 57,0 C 7,4 H 5,1 N 13,0 Cl
Gef. : 56,8 C 7,2 H 5,1 N 12,6 Cl

Beispiel XI

1-(2-Carboäthoxy-4,5-dimethyl-pyrrol-3-oxy)-3-chlorpropan

Gemäß Beispiel VII erhält man aus 18,3 g 3-Hydroxy-4,5-dimethyl-2-pyrrolcarbonsäureäthylester, 25 g 1,3-Bromchlorpropan und 18 g wasserfreiem Kaliumcarbonat 11,2 g 1-(2-Carboäthoxy-4,5-dimethylpyrrol-3-oxy)-3-chlorpropan vom Schmp. 60 bis 63 °C.

$C_{12}H_{18}NO_3Cl$ (259,6)
Ber. :55,5 C 6,9 H 5,4 N 13,7 Cl
Gef. :55,2 C 6,7 H 5,1 N 13,5 Cl

Beispiel XII

1-(2-Carboäthoxy-4,5-dimethyl-pyrrol-3-oxy)-2-chloräthan

Gemäß Beispiel VII erhält man aus 18,3 g 3-Hydroxy-4,5-dimethyl-2-pyrrolcarbonsäureäthylester, 20, 5 g 1,2-Bromchloräthan und 18 g wasserfreiem Kaliumcarbonat, 10 g 1-(2-Carboäthoxy-4,5-dimethyl-pyrrol-3-oxy)-2-chloräthan vom Schmp. 81 bis 83 °C.

$C_{11}H_{16}NO_3Cl$ (245,6)
Ber. : 53,8 C 6,5 H 5,7 N 14,4 Cl
Gef. : 54,2 C 6,5 H 5,9 N 14,7 Cl

Beispiel XIII

4,0 g 1-(2-Carboäthoxy-4,5-dimethyl-pyrrol-3-oxy)-2,3-epoxypropan werden in einem Gemisch aus 20 ml Äthanol und 15 ml einer ca. 4 N ätherischen Salzsäure gelöst. Nach eintägigem Stehen werden die flüchtigen Bestandteile abdestilliert und der Eindampfrückstand an Kieselgel mit Methylenchlorid chromatographiert. Man erhält 2,7 g NMR-spektroskopisch reines 1-(2-Carboäthoxy-4,5-dimethylpyrrol-3-oxy)-3-chlor-propanol-2.

$^1H$-NMR-Spektrum ($CDCl_3$, TMS intern) :
$\tau$ = 1,4 (breites s, 1H) ; 5,5 (s, OH) ; 5,7 (g 2H, J=4,5 Hz) ; 5,9 (m, 3H) ; 6,4 (m, 2H) ; 7,88 (s, 3H) ; 8,1 (s, 3H) ; 8,7 (t, 3H, J=4,5 Hz).

II. Erfindungsgemäße Verbindungen

Beispiel 1

24 g 1-(2-Carboäthoxy-4,5-dimethyl-pyrrol-3-oxy)-2,3-epoxy-propan und 18 g 4-(2-Pyridyl)-piperidin-4-ol werden in 200 ml Äthanol 4 Stunden auf Rückflußtemperatur erhitzt. Der nach dem Abdestillieren von Äthanol verbleibende Rückstand wird in wenig Methanol gelöst und tropfenweise mit ätherischer Salzsäure versetzt. Das ausgefallene Kristallisat wird abgesaugt, mit Äther gewaschen und getrocknet. Man erhält 29 g 3-[2-Hydroxy-3-(4-α-Pyridyl-4-hydroxy-piperidino)-propoxy]-4,5-dimethyl-pyrrol-2-carbonsäureäthylester-dihydrochlorid vom Schmp. 227 bis 229 °C.

$C_{22}H_{33}N_3O_5Cl_2$ (490,1)
Ber.: 53,8 C 6,7 H 8,6 N 14,5 Cl
Gef.: 53,5 C 6,7 H 8,5 N 14,3 Cl

## Beispiel 2

55 g 1-(2-Carboäthoxy-4,5-dimethyl-pyrrol-3-oxy)-2,3-epoxypropan und 41 g 4-Hydroxy-4-phenylpiperidin werden in 600 ml Äthanol 8 Stunden auf Rückflußtemperatur erhitzt. Der nach dem Abdestillieren von Äthanol verbleibende Rückstand wird in wenig Methanol gelöst und tropfenweise mit ätherischer Salzsäure versetzt. Das ausgefallene Kristallisat wird abgesaugt, mit Äther gewaschen und getrocknet. Man erhält 81 g 3-[2-Hydroxy-3-(4-phenyl-4-hydroxy-piperidino)-propoxy]-4,5-dimethyl-pyrrol-2-carbonsäureäthylester-hydrochlorid vom Schmp. 211 bis 212 °C.

$C_{23}H_{33}N_2O_5Cl$ (454,0)
Ber.: 61,0 C 7,3 H 17,7 O 6,2 N 7,8 Cl
Gef.: 61,2 C 7,1 H 17,9 O 5,9 N

## Beispiel 3

50 g 1-(2-Carboäthoxy-4,5-dimethyl-pyrrol-3-oxy)-3-chlorpropan, 35 g 4-Hydroxy-4-phenyl-piperidin und 50 g Natriumcarbonat werden in 300 ml N,N-Dimethylformamid 20 Stunden auf 100 °C erhitzt. Nach dem Erkalten wird zwischen Wasser und Methylenchlorid verteilt und nach dem Eindampfen der organischen Phase im Vakuum verbleibende Rückstand in wenig Methanol gelöst und tropfenweise mit ätherischer Salzsäure versetzt. Das ausgefallene Kristallat wird abgesaugt, mit Äther gewaschen und getrocknet. Man erhält 56 g 3-[3-(4-Phenyl-4-hydroxy-piperidino)-propoxy]-4,5-dimethyl-pyrrol-2-carbonsäureäthylester-hydrochlorid vom Schmp. 165 bis 169 °C.

$C_{23}H_{33}N_2O_5Cl$ (454,0)
Ber.: 61,0 C 7,3 H 17,7 O 6,2 N 7,8 Cl
Gef.: 61,2 C 7,1 H 17,9 O 5,9 N

## Beispiel 4

In einem Autoklaven werden 6,0 g 1-(2-Carboäthoxy-4,5-dimethyl-pyrrol-3-oxy)-3-chlor-propanol-2 und 3,5 g 4-Hydroxy-4-phenylpiperidin in 150 ml Dioxan 15 Stunden auf 120 °C erhitzt. Nach dem Abdestillieren der flüchtigen Anteile im Vakuum wird der hochviskose Rohaustrag in Äther-2 N Schwefelsäure verteilt, die Wasserphase vorsichtig mit 4N Natronlauge alkalisch gestellt und anschließend mit Äther extrahiert. Nach dem Trocknen der organischen Phase über Magnesiumsulfat wird vom Lösungsmittel befreit und der verbleibende Rückstand, wie in Beispiel 2 beschrieben, in wenig Methanol gelöst und mit ätherischer Salzsäure in 3,2 g 3-[2-Hydroxy-3-(4-phenyl-4-hydroxy-piperidino)-propoxy]-4,5-dimethylpyrrol-2-carbonsäureäthylester-hydrochlorid vom Schmp. 210 bis 212 °C überführt.

Die in Tabelle 2 angegebenen Verbindungen werden gemäß Beispiel 2 aus den entsprechenden Glycidäthern und den 4-Hydroxy-4-phenyl-piperidinen oder gemäß Beispiel 3 aus den entsprechenden 1-(2-Carboäthoxy-4,5-dimethylpyrrol-3-oxy)-chloralkanen und den 4-Hydroxy-4-phenyl-piperidinen erhalten.

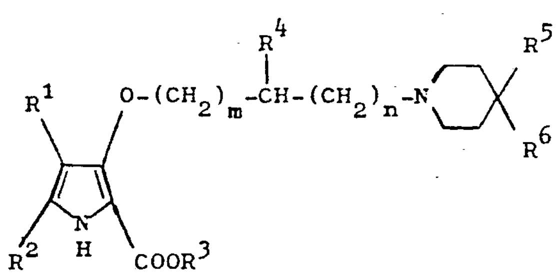

Tabelle 2

| Beispiel Nummer | $R^1$ | $R^2$ | $R^3$ | $R^4$ | $R^5$ | $R^6$ | m | n | Salzform | Schmp. °C |
|---|---|---|---|---|---|---|---|---|---|---|
| 5 | $CH_3$ | $CH_3$ | $CH_3$ | H | OH | 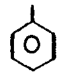 | 1 | 1 | — | 140-141 |

Tabelle 2 (Fortsetzung)

| Beispiel Nummer | $R^1$ | $R^2$ | $R^3$ | $R^4$ | $R^5$ | $R^6$ | m | n | Salzform | Schmp. °C |
|---|---|---|---|---|---|---|---|---|---|---|
| 6 | $CH_3$ | $CH_3$ | $CH_3$ | OH | OH | 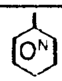 | 1 | 1 | 2 HCl | 220-221 |
| 7 | $CH_3$ | $CH_3$ | $CH_3$ | H | OH | 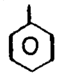 | 1 | 0 | — | 122-123 |
| 8 | $CH_3$ | $CH_3$ | $C_2H_5$ | H | OH | 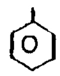 | 1 | 2 | HCl | 184-185 |
| 9 | $CH_3$ | $CH_3$ | $C_2H_5$ | H | OH | 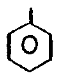 | 1 | 0 | HCl | 227-228 |
| 10 | $CH_3$ | $CH_3$ | $CH_3$ | H | OH | 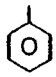 | 1 | 2 | — | 136-137 |
| 11 | $CH_3$ | $CH_3$ | $C_2H_5$ | OH | H | 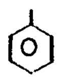 | 1 | 1 | HCl | 244-245 |
| 12 | $CH_3$ | $CH_3$ | $C_2H_5$ | OH | OH | 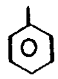 | 1 | 1 | HCl | 216-218 |
| 13 | $CH_3$ | $CH_3$ | $C_2H_5$ | OH | OH | 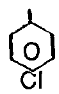 | 1 | 1 | 1/2 HOOC COOH | 214-216 |
| 14 | $CH_3$ | $CH_3$ | $CH_3$ | OH | OH | 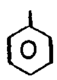 | 1 | 1 | HCl | 216-217 |
| 15 | n-$C_4H_9$ | $CH_3$ | $C_2H_5$ | OH | OH | 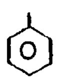 | 1 | 1 | $(COOH)_2$ | 124-126 |

| Beispiel | Analysen | Name |
|---|---|---|
| 5 | Ber.: 68,4 C 7,8 H 7,3 N<br>Gef.: 68,1 C 7,7 H 7,4 N | 3-[3-(4-Phenyl-4-hydroxy-piperidino)-propoxy]-4,5-dimethylpyrrol-2-carbonsäuremethylester |
| 6 | Ber.: 53,0 C 6,6 H 8,8 N 14,9 Cl<br>Gef.: 52,9 C 6,6 H 8,7 N 15,2 Cl | 3-[2-Hydroxy-3-(4-α-pyridyl-4-hydroxy-piperidino)-propoxy]-4,5-dimethylpyrrol-2-carbonsäuremethylester |
| 7 | Ber.: 67,7 C 7,6 H 7,5 N<br>Gef.: 67,5 C 7,5 H 7,7 N | 3-[2-(4-Phenyl-4-hydroxy-piperidino)-äthoxy]-4,5-dimethylpyrrol-2-carbonsäuremethylester |
| 8 | Ber.: 63,9 C 7,8 H 6,2 N 7,9 Cl<br>Gef.: 63,6 C 7,6 H 6,2 N 7,8 Cl | 3-[4-(4-Phenyl-4-hydroxy-piperidino)-butoxy]-4,5-dimethylpyrrol-2-carbonsäureäthylester |
| 9 | Ber.: 62,5 C 7,4 H 6,6 N 8,4 Cl<br>Gef.: 62,1 C 7,2 H 6,6 N 8,3 Cl | 3-[2-(4-Phenyl-4-hydroxy-piperidino)-äthoxy]-4,5-dimethylpyrrol-2-carbonsäureäthylester |

(Fortsetzung)

| Beispiel | Analysen | | | | | Name |
|---|---|---|---|---|---|---|
| 10 | Ber. : 69,0 C<br>Gef. : 68,7 C | 8,1 H<br>7,7 H | 7,0 N<br>7,0 N | | | 3-[4-(4-Phenyl-4-hydroxy-piperidino)-butoxy]-4,5-dimethylpyrrol-2-carbonsäuremethylester |
| 11 | Ber. : 63,2 C<br>Gef. : 62,5 C | 7,6 H<br>7,6 H | 14,7 O<br>14,7 O | 6,4 N<br>6,4 N | 8,1 Cl<br>8,3 Cl | 3-[2-Hydroxy-3-(4-phenyl-piperidino)-propoxy]-4,5-dimethylpyrrol-2-carbonsäureäthylester |
| 12 | Ber. : 58,7 C<br>Gef. : 58,2 C | 6,9 H<br>6,6 H | 6,0 N<br>5,6 N | 4,0 F<br>3,8 F | 7,3 Cl<br>7,7 Cl | 3-[2-Hydroxy-3-(4-(p-Fluorphenyl)-4-hydroxy-piperidino)-propoxy]-4,5-dimethylpyrrol-2-carbonsäureäthylester |
| 13 | Ber. : 59,0 C<br>Gef. : 58,5 C | 6,5 H<br>6,3 H | 5,5 N<br>5,5 N | 7,0 Cl<br>6,9 Cl | | 3-[2-Hydroxy-3-(4-(p-chlorphenyl)-4-hydroxy-piperidino)-propoxy]-4,5-dimethylpyrrol-2-carbonsäureäthylester |
| 14 | Ber. : 60,2 C<br>Gef. : 60,0 C | 7,1 H<br>6,9 H | 6,4 N<br>6,5 N | 8,1 Cl<br>8,2 Cl | | 3-[2-Hydroxy-3-(4-phenyl-4-hydroxypiperidino)-propoxy]-4,5-dimethylpyrrol-2-carbonsäuremethylester |
| 15 | Ber. : 61,3 C<br>Gef. : 61,6 C | 7,4 H<br>7,2 H | 5,1 N<br>5,3 N | | | 3-[2-Hydroxy-3-(4-phenyl-4-hydroxypiperidino)-propoxy]-4-n-butyl-5-methyl-pyrrol-2-carbonsäureäthylester |

III. Formulierungsbeispiele, die in üblicher Weise hergestellt werden :

1. Tabletten :

| | |
|---|---|
| a) Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |

| | |
|---|---|
| b) Ein Wirkstoff der Formel I | 20 mg |
| Lactose | 178 mg |
| Avicel | 80 mg |
| Polywachs 6 000 | 20 mg |
| Magnesiumstearat | 2 mg |

| | |
|---|---|
| c) Ein Wirkstoff der Formel I | 50 mg |
| Polyvinylpyrrolidon (mittl.M.G. 25 000) | 170 mg |
| Polyäthylenglykol (mittl.M.G. 4 000) | 14 mg |
| Hydroxypropylmethylcellulose | 40 mg |
| Talkum | 4 mg |
| Magnesiumstearat | 2 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50 °C getrocknet. Dieses Granulat wird mit Polyäthylenglykol (mittl. M.G. 4 000), Hydroxypropylmethylcellulose, Talkum und Magnesiumstearat vermischt und zu Tabletten á 280 mg verpreßt.

2. Beispiel für Dragees

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50 °C getrocknet und nochmals durch Sieb 1,0 mm gerieben. Das so erhaltene Granulat wird mit Magnesiumstearat gemischt und zu

Drageekernen verpreßt. Die erhaltenen Drageekerne werden in üblicher Weise mit einer Hülle überzogen, die im wesentlichen aus Zucker und Talkum besteht.

3. Kapselformulierung

| | |
|---|---|
| Ein Wirkstoff der Formel I | 5 mg |
| Magnesiumstearat | 2,0 mg |
| Milchzucker | 19,3 mg |

4. Injektionslösung

| | |
|---|---|
| Ein Wirkstoff der Formel I | 10 mg |
| Natriumchlorid | 9 mg |
| destilliertes Wasser, q.s. auf 1,0 ml | |

**Ansprüche**

1. Verbindungen der allgemeinen Formel I

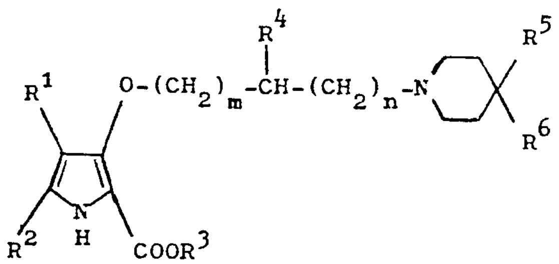

(I)

in der $R^1$ einen Alkylrest mit 1 bis 4 C-Atomen, $R^2$ Methyl, $R^3$ Methyl oder Äthyl, $R^4$ ein Wasserstoffatom oder eine Hydroxylgruppe, $R^5$ eine Hydroxylgruppe und $R^6$ α-Pyridyl oder einen Phenylring, der gegebenenfalls durch ein Fluor-, Chlor- oder Bromatom substituiert ist, bedeuten, wobei m und n 1 oder 2 oder, wenn $R^4$ ein Wasserstoffatom darstellt, zusätzlich m 1 und n 0 sind und ihre physiologisch verträglichen Säureadditionssalze.

2. 3-[2-Hydroxy-3-(4-α-Pyridyl-4-hydroxy-piperidino)-propoxy]-4,5-dimethyl-pyrrol-2-carbonsäureäthylester und dessen physiologisch verträglichen Säureadditionssalze.

3. 3-[2-Hydroxy-3-(4-phenyl-4-hydroxy-piperidino)-propoxy]-4,5-dimethylpyrrol-2-carbonsäureäthylester und dessen physiologisch verträglichen Säureadditionssalze.

4. 3-[2-Hydroxy-3-(4-α-pyridyl-4-hydroxy-piperidino)-propoxy]-4,5-dimethylpyrrol-2-carbonsäuremethylester und dessen physiologisch verträglichen Säureadditionssalze.

5. 3-[2-(4-phenyl-4-hydroxy-piperidino)-äthoxy]-4,5-dimethylpyrrol-2-carbonsäureäthylester und dessen physiologisch verträglichen Säureadditionssalze.

6. 3-[4-(4-Phenyl-4-hydroxy-piperidino)-butoxy]-4,5-dimethylpyrrol-2-carbonsäuremethylester und dessen physiologisch verträglichen Säureadditionssalze.

7. 3-[2-Hydroxy-3-(4-phenyl-4-hydroxy-piperidino)-propoxy]-4,5-dimethylpyrrol-2-carbonsäuremethylester und dessen physiologisch verträglichen Säureadditionssalze.

8. Verfahren zur Herstellung von Verbindungen der Formel I nach Anspruch 1, dadurch gekennzeichnet, daß man ein Carboalkoxypyrrol der Formel (II)

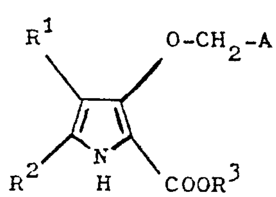

(II)

in der $R^1$, $R^2$ und $R^3$ die für Formel I angegebenen Bedeutungen haben und A den Rest

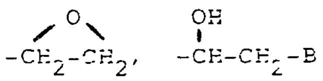 oder $-(CH_2)_l-B$,

wobei B für eine nukleofuge Abgangsgruppe und l für eine ganze Zahl von 1 bis 4 stehen, bedeutet, mit einem Piperidin-Derivat der allgemeinen Formel III

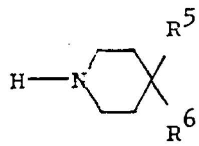

(III)

in der $R^5$ und $R^6$ die für Formel I angegebenen Bedeutungen haben, zweckmäßigerweise in einem Lösungsmittel bei Temperaturen von 10 bis 120 °C und ggf. in Gegenwart eines säurebindenden Mittels in an sich bekannter Weise umsetzt und die erhaltene Verbindung ggf. in das Säureadditionssalz einer physiologisch verträglichen Säure überführt.

9. Therapeutisches Mittel, gekennzeichnet durch einen Gehalt an einer Verbindung der Formel I nach Anspruch 1 oder ihres physiologisch verträglichen Säureadditionssalzes als Wirkstoff neben üblichen Trägerstoffen und Verdünnungsmitteln.

10. Verbindung der Formel I nach Anspruch 1, zur Verwendung bei der Pharmakotheraphie von Herzrhythmusstörungen.

**Claims**

1. A compound of the general formula I

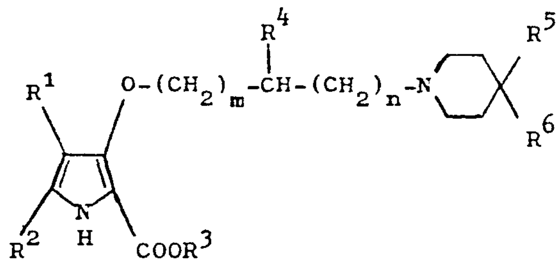

(I)

where $R^1$ is alkyl of 1 to 4 carbon atoms, $R^2$ is methyl, $R^3$ is methyl or ethyl, $R^4$ is hydrogen or hydroxyl, $R^5$ is hydroxyl, $R^6$ is α-pyridyl or a phenyl ring optionally substituted by fluorine, chlorine or bromine, and m and n are 1 to 2 or, if $R^4$ is hydrogen, m is 1 and n is 0, and its physiologically tolerated addition salts with acids.

2. 3-[2-Hydroxy-3-(4-α-pyridyl-4-hydroxy-piperidino)-propoxy]-4,5-dimethyl-pyrrole-2-carboxylic acid ethyl ester and its physiologically tolerated addition salts with acids.

3. 3-[2-Hydroxy-3-(4-phenyl-4-hydroxy-piperidino)-propoxy]-4,5-dimethyl-pyrrole-2-carboxylic acid ethyl ester and its physiologically tolerated addition salts with acids.

4. 3-[2-Hydroxy-3-(4-α-pyridyl-4-hydroxy-piperidino)-propoxy]-4,5-dimethyl-pyrrole-2-carboxylic acid methyl ester and its physiologically tolerated addition salts with acids.

5. 3-[2-(4-Phenyl-4-hydroxy-piperidino)-ethoxy]-4,5-dimethyl-pyrrole-2-carboxylic acid ethyl ester and its physiologically tolerated addition salts with acids.

6. 3-[4-(4-Phenyl-4-hydroxy-piperidino)-butoxy]-4,5-dimethyl-pyrrole-2-carboxylic acid methyl ester and its physiologically tolerated addition salts with acids.

7. 3-[2-Hydroxy-3-(4-phenyl-4-hydroxy-piperidino)-propoxy]-4,5-dimethyl-pyrrole-2-carboxylic acid methyl ester and its physiologically tolerated addition salts with acids.

8. A process for the preparation of a compound of the formula I as claimed in claim 1, wherein a carboalkoxypyrrole of the formula II

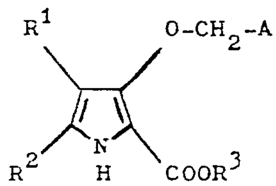

(II)

where $R^1$, $R^2$ and $R^3$ have the meanings given for formula I and A is

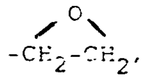
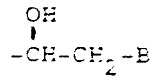
or $(CH_2)_l-B$,

where B is a nucleofugic leaving group and l is an integer from 1 to 4, is reacted in a conventional manner with a piperidine derivative of the general formula III

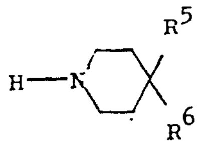
(III)

where $R^5$ and $R^6$ have the meanings given for formula I, advantageously in a solvent, at from 10 to 120 °C, and in the presence or absence of an acid acceptor, and, if desired, the resulting compound is converted into its addition salt with a physiologically tolerated acid.

9. A therapeutic agent which contains a compound of the formula I as claimed in claim 1, or a physiologically tolerated acid addition salt thereof, as the active compound, together with conventional carriers and diluents.

10. A compound of the formula I as claimed in claim 1 for use in the pharmacotherapy of cardiac arrhythmias.

**Revendications**

1. Composés de formule générale I

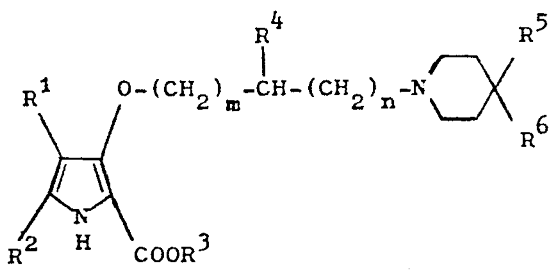
(I)

dans laquelle $R^1$ représente un reste alkyle ayant 1 à 4 atomes C, $R^2$ méthyle, $R^3$ méthyle ou éthyle, $R^4$ un atome d'hydrogène ou un groupe hydroxyle, $R^5$ un groupe hydroxyle et $R^6$ α-pyridyle ou un noyau phényle, qui est éventuellement substitué par un atome de fluor, chlore ou brome, *m* et *n* représentant 1 ou 2, ou bien, lorsque $R^4$ représente un atome d'hydrogène, en outre *m* est 1 et *n* est 0, et leurs sels d'addition d'acide compatibles physiologiquement.

2. Ester éthylique d'acide 3-[2-hydroxy-3-(4-α-pyridyl-4-hydroxy-pipéridino)-propoxy]-4,5-diméthyl-pyrrol-2-carboxylique et ses sels d'addition d'acide compatible physiologiquement.

3. Ester éthylique d'acide 3-[2-hydroxy-3-(4-phényl-4-hydroxy-pipéridino)-propoxy]-4,5-diméthylpyrrol-2-carboxylique et ses sels d'addition d'acide compatible physiologiquement.

4. Ester méthylique d'acide 3-[2-hydroxy-3-(4-α-pyridyl-4-hydroxy-pipéridino)-propoxy]-4,5-diméthyl-pyrrol-2-carboxylique et ses sels d'addition d'acide compatibles physiologiquement.

5. Ester éthylique d'acide 3-[2-(4-phényl-4-hydroxy-pipéridino)-éthoxy]-4,5-diméthylpyrrol-2-carboxylique et ses sels d'addition d'acide compatibles physiologiquement.

6. Ester méthylique d'acide 3-[4-(4-phényl-4-hydroxy-pipéridino)-butoxy]-4,5-diméthylpyrrol-2-carboxylique et ses sels d'addition d'acide compatibles physiologiquement.

7. Ester méthylique d'acide 3-[2-hydroxy-3-(4-phényl-4-hydroxy-pipéridino)-propoxy]-4,5-diméthyl-pyrrol-2-carboxylique et ses sels d'addition d'acide compatibles physiologiquement.

8. Procédé de préparation de composés de formule I selon la revendication 1, caractérisé par le fait qu'on fait réagir, de manière connue en soi, de préférence dans un solvant, à des températures de 10 à 120 °C et éventuellement en présence d'un agent liant les acides, un carboalcoxypyrrol de formule (II)

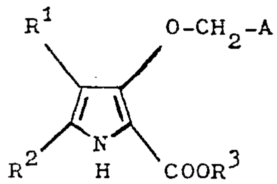

(II)

dans laquelle $R^1$, $R^2$ et $R^3$ ont les significations données pour la formule I et A le reste

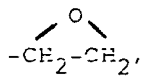 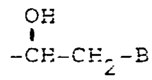 ou $-(CH_2)_l-B$,

B représentant un groupe de départ nucléofuge et l un nombre entier de 1 à 4, avec un dérivé de pipéridine de formule générale III

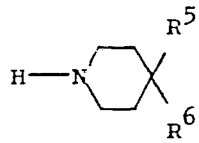

(III)

dans laquelle $R^5$ et $R^6$ ont les significations données pour la formule I.

9. Agent thérapeutique, caractérisé par le fait qu'il contient, comme principe actif, un composé de formule I selon la revendication 1 ou son sel d'addition d'acide compatible physiologiquement, outre des supports et diluants usuels.

10. Composé de formule I selon la revendication 1, pour l'utilisation en pharmacothérapie des troubles du rythme cardiaque.